# EUROPÄISCHE PATENTANMELDUNG

(11) **EP 2 868 652 A1**
(43) Veröffentlichungstag der Anmeldung: **06.05.2015**
(21) Anmeldenummer: 13191498.8
(22) Anmeldetag: 05.11.2013
(51) Int. Cl.: C07C 209/52, C07C 209/68, C07C 213/00, C07D 295/023, C07D 295/027, C07D 295/03, C07B 43/04, C07C 253/30

(54) **Katalytische Hydrierung zur Herstellung von Aminen aus Amidacetalen, Keten-N,O-acetalen oder Esterimiden**

(71) Anmelder: Evonik Industries AG, 45128 Essen (DE)
(72) Erfinder: Kadyrov, Renat, Dr., 60389 Frankfurt (DE)

(57) **Zusammenfassung**

Die vorliegende Erfindung betrifft ein Verfahren zur Herstellung von Aminen, umfassend die folgenden Schritte: Umsetzung eines (i) Amidacetals der allgemeinen Formel (I), oder (ii) Keten-N,0-acetals der allgemeinen Formel (II), oder (iii) Esterimids der allgemeinen Formel (III) mit H₂ in Gegenwart eines Hydrierkatalysators, wobei Katalysator und Amidacetal oder Keten-N,0-acetal oder Esterimid in einem Mol-Verhältnis von 1:10 bis 1:100.000 eingesetzt werden und wobei ein Wasserstoffdruck von 0,1 bar bis 200 bar eingestellt wird und wobei eine Temperatur im Bereich von 0°C bis 250°C eingestellt wird.

## Beschreibung

Die vorliegende Erfindung betrifft ein Verfahren zur Herstellung von Aminen, wobei ein Amidacetal, Keten-N,O-acetal oder Esterimid mit H₂ in Gegenwart eines Hydrierkatalysators, wobei Katalysator und Amidacetal oder Keten-N,O-acetal oder Esterimid in einem Mol-Verhältnis von 1:10 bis 1:100.000 eingesetzt werden und wobei ein Wasserstoffdruck von 0,1 bar bis 200 bar eingestellt wird und wobei eine Temperatur im Bereich von 0°C bis 250°C eingestellt wird. Die Amidacetale, Keten-N,O-acetale oder Esterimide können nach diesem Verfahren hochselektiv und unter sehr milden Bedingungen zu Aminen hydriert werden.

Reduktion von Amiden gehört mit zu den wichtigsten Methoden für die Herstellung von Aminen. Das klassische Verfahren basiert auf der Reduktion durch komplexe Hydride, wobei jedoch stöchiometrische Mengen an Hydrid erforderlich sind und die Selektivität relativ gering ist. Die Entwicklung der katalytischen Reduktion mit Wasserstoff bleibt bis heute eine der größten Herausforderungen. In der Literatur sind derartige Hydrierungen bekannt, jedoch sind große Mengen (15 mol % und mehr) an Katalysator, sehr hohe Drücke und Temperaturen über 200°C notwendig, um brauchbare Ausbeuten zu erzielen (S. Nishimura, Handbook of Heterogeneous Catalytic Hydrogenation for Organic Synthesis 2001, pp. 406-411, Wiley, N.Y.,). Kürzlich wurde über die Hydrierung von tertiären und sekundären Amiden zu Aminen bei 120-160°C über einen bimetallischen Pd-Re-Katalysator berichten (M. Stein , B. Breit, Angew. Chem. 2013, 125, 2287-2290). Trotz etwas milderer Bedingungen wird kaum eine funktionelle Gruppe toleriert, sogar olefinische Doppelbindungen und aromatische Ringe werden durchhydriert.
Über die Hydrogenolyse von Amidacetalen und Iminoethern ist dagegen kaum etwas bekannt. Das Patent DE 604277C (W. Klempt, F. Brodkorb) beschreibt, dass die Hydrochloride der primären Iminoether durch Hydrieren in Gegenwart von Adams-Katalysator (Platinoxid) mit hohen Ausbeuten in die primären Amine umgewandelt werden können. Eine Wiederholung von Versuchsprotokoll 1 aus der Patentschrift wurde durchgeführt, und die Produkte wurden mit modernen Methoden (GC-MS und NMR) charakterisiert. Die Produktanalyse zeigte jedoch, dass unter den in DE604277C angegebenen Bedingungen zum größten Teil das Bis(dicyclohexylmethyl)amin entsteht, ein sekundäres Amin mit durchhydrierten Benzolringen (Beispiel 1 im Kapitel Ausführungsbeispiele).
In den letzten 50 Jahren nahm die Entwicklung von synthetischen Methoden zur Herstellung von Amidacetalen und Imidoestern einen breiten Aufschwung. Bekannterweise zählen heute die Amidacetale und Imidoester zu den leicht zugänglichen Substanzklassen (G. Simchen, Methoden Org. Chem., 1985, E5/1, S. 1-192 (Houben-Weyl); N. Nakajima, M. Ubukata, Science of Synthesis 2005, Vol. 22, pp. 343-360, Thieme Chemistry Stuttgart).

Die Aufgabe der vorliegenden Erfindung war es daher, ein Verfahren zur Herstellung von Aminen mittels katalytischer Hydrierung von Säureamidacetalen, Keten-N,O-acetalen und Säureesterimiden mit Wasserstoff zur Verfügung zu stellen.

Überraschenderweise wurde nun gefunden, dass Amidacetale, Keten-N,O-acetale und Esterimide unter sehr milden Bedingungen in Gegenwart von üblichen Hydrierkatalysatoren zu Aminen hydriert werden können. Dabei werden unterschiedlichste funktionelle Gruppen toleriert, unter anderem bleiben Nitrile, Carboxyl- und Phosphon-Gruppen erhalten.

Die technische Aufgabe wird gelöst durch ein Verfahren zur Herstellung von Aminen umfassend die folgenden Schritte: a. Umsetzung eines
i. Amidacetals der allgemeinen Formel (I) 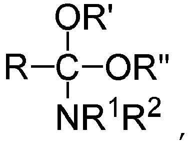 wobei
   R ausgewählt wird aus der Gruppe bestehend aus H, (C₁-C₂₄)-Alkyl, (C₃-C₂₀)-Cycloalkyl, (C₂-C₁₃) - Heterocycloalkyl, (C₆-C₁₄)-Aryl oder (C₃-C₁₃)-Heteroaryl, CN, COO-(C₁-C₁₈)-Alkyl, CONH-(C₁-C₁₈)-Alkyl, CF₃;
   R' und R" unabhängig voneinander ausgewählt werden aus der Gruppe (C₁-C₂₄)-Alkyl, (C₃-C₂₀) -Cycloalkyl, (C₂-C₁₃)-Heterocycloalkyl; und
   R¹ und R² unabhängig voneinander ausgewählt werden aus der Gruppe bestehend aus H, (C₁-C₂₄)-Alkyl, (C₃-C₈)-Cycloalkyl, (C₂-C₇)-Heterocycloalkyl, (C₆-C₁₄)-Aryl und (C₃-C₁₃)-Heteroaryl;
   wobei auch aus jeweils zwei Resten ausgewählt aus R, R¹, R², R' und R" gemeinsam eine (C₂-C₈)-AlkylenBrücke gebildet werden kann, so dass ein Ring mit insgesamt 4-11 Ringatomen entsteht; oder
ii. Keten-N,O-acetals der allgemeinen Formel (II) 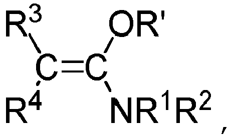 wobei
   R' ausgewählt wird aus der Gruppe bestehend aus (C₁-C₂₄)-Alkyl, (C₃-C₂₀)-Cycloalkyl, (C₂-C₁₃) - Heterocycloalkyl; und
   R¹, R² unabhängig voneinander ausgewählt werden aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl und (C₃-C₁₃)-Heteroaryl;
   R³ und R⁴ unabhängig voneinander ausgewählt werden aus der Gruppe bestehend aus H, (C₁-C₂₄)-Alkyl, (C₃-C₂₀) - Cycloalkyl, (C₂-C₁₃)-Heterocycloalkyl, (C₆-C₁₄)-Aryl und (C₃-C₁₃) -Heteroaryl, CN, COO- (C₁-C₁₈) -Alkyl, CONH- (C₁-C₁₈)-Alkyl und CF₃,
   wobei auch aus jeweils zwei Resten ausgewählt aus R¹, R², R³, R⁴ und R' gemeinsam eine (C₂-C₈)-Alkylen-Brücke gebildet werden kann, so dass ein Ring mit insgesamt 3-11 Ringatomen entsteht;
   oder
iii. Esterimids der allgemeinen Formel (III) 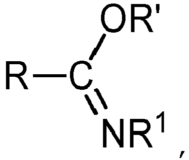 wobei
   R' ausgewählt wird aus der Gruppe bestehend aus (C₁-C₂₄)-Alkyl, (C₃-C₂₀)-Cycloalkyl, (C₂-C₁₃)- Heterocycloalkyl;
   R ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃) -Heteroaryl, CN, COO- (C₁-C₁₈)-Alkyl, CONH- (C₁-C₁₈)-Alkyl, CF₃;
   R¹ ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄) -Aryl oder (C₃-C₁₃)-Heteroaryl;
   wobei auch aus jeweils zwei Resten ausgewählt aus R, R¹ und R' gemeinsam eine (C₂-C₈)-Alkylen-Brücke gebildet werden kann, so dass ein Ring mit insgesamt 4-11 Ringatomen entsteht;
mit H₂ in Gegenwart eines Hydrierkatalysators, wobei Katalysator und Amidacetal oder Keten-N,O-acetal oder Esterimid in einem Mol-Verhältnis von 1:10 bis 1:100.000 eingesetzt werden und wobei ein Wasserstoffdruck von 0,1 bar bis 200 bar eingestellt wird und wobei eine Temperatur im Bereich von 0°C bis 250°C eingestellt wird.

Im Sinne der vorliegenden Erfindung bezieht sich der Begriff Amidacetal auf alle Arten von Amidacetalen der allgemeinen Formel (I), unabhängig davon, ob es Acetale von Mono- oder Diamiden, ob es cyclische Amidacetale oder acyclische Amidacetale sind. 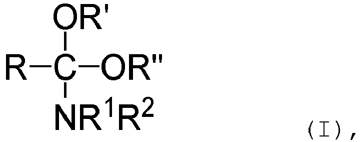 wobei
R ausgewählt wird aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃) -Heteroaryl, CN, COO- (C₁-C₁₈) -Alkyl, CONH- (C₁-C₁₈) -Alkyl, CF₃;
R' und R" unabhängig voneinander ausgewählt werden aus der Gruppe (C₁-C₂₄)-Alkyl, (C₃-C₂₀) -Cycloalkyl, (C₂-C₁₃)-Heterocycloalkyl; und
R¹ und R² unabhängig voneinander ausgewählt werden aus der Gruppe bestehend aus H, (C₁-C₂₄)-Alkyl, (C₃-C₈)-Cycloalkyl, (C₂-C₇)-Heterocycloalkyl, (C₆-C₁₄)-Aryl und (C₃-C₁₃)-Heteroaryl;
wobei auch aus jeweils zwei Resten ausgewählt aus R, R¹, R², R' und R" gemeinsam eine (C₂-C₈)-Alkylen-Brücke gebildet werden kann, so dass ein Ring mit insgesamt 4-11 Ringatomen entsteht.
R ist bevorzugt ausgewählt aus der Gruppe bestehend aus H, Methyl, Ethyl, n-Propyl, n-Butyl, iso-Butyl, n-Pentyl, n-Hexyl, Cyclohexyl, n-Heptyl, n-Octyl, Phenyl, Pyridyl, Naphthyl.
R' sind R" bevorzugt ausgewählt aus der Gruppe bestehend aus Methyl, Ethyl, Benzyl, 1,2-Ethylen;
R¹ und R² sind bevorzugt ausgewählt aus der Gruppe bestehend aus Methyl, Ethyl, n-Propyl, iso-Propyl, n-Butyl, iso-Butyl, n-Pentyl, n-Hexyl, n-Heptyl, n-Octyl, Butandi-1,4-yl, Pentandi-1,5-yl, Hexandi-1,6-yl und Phenyl.
Ein Ring wird bevorzugt zwischen den Resten R und R¹ oder R¹ und R' gebildet, wobei der Ring insgesamt bevorzugt 5, 6 oder 7 Ringatome aufweist. Bevorzugt gebildete Ringe sind Pyrrolidin, Piperidin, Morpholin, Piperazin, Homopiperidin und Homopiperazin und deren Derivate.

Im Sinne der vorliegenden Erfindung bezieht sich der Begriff Keten-N,O-acetale auf Verbindungen der allgemeinen Formel (II) 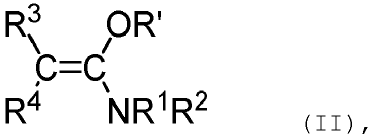 wobei
R' ausgewählt wird aus der Gruppe bestehend aus (C₁-C₂₄)-Alkyl, (C₃-C₂₀)-Cycloalkyl, (C₂-C₁₃) -Heterocycloalkyl; und R¹, R² unabhängig voneinander ausgewählt werden aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl und (C₃-C₁₃)-Heteroaryl;
R³ und R⁴ unabhängig voneinander ausgewählt werden aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl und (C₃-C₁₃)-Heteroaryl, CN, COO-(C₁-C₁₈)-Alkyl, CONH-(C₁-C₁₈)-Alkyl und CF₃,
wobei auch aus jeweils zwei Resten ausgewählt aus R¹, R², R³, R⁴ und R' gemeinsam eine (C₂-C₈)-Alkylen-Brücke gebildet werden kann, so dass ein Ring mit insgesamt 3-11 Ringatomen entsteht.
R' ist bevorzugt ausgewählt aus der Gruppe bestehend aus Methyl und Ethyl;
R¹ und R² sind bevorzugt ausgewählt aus der Gruppe bestehend aus Methyl, Ethyl, n-Propyl, iso-Propyl, n-Butyl, iso-Butyl, n-Pentyl, n-Hexyl, n-Heptyl, n-Octyl, Butandi-1,4-yl, Pentandi-1,5-yl, Hexandi-1,6-yl und Phenyl.
R³ und R⁴ sind bevorzugt ausgewählt aus der Gruppe bestehend aus H, (C₆-C₁₄)-Aryl, CN, COO-(C₁-C₁₈)-Alkyl, CONH-(C₁-C₁₈)-Alkyl und CF₃.
Ein Ring wird bevorzugt zwischen den Resten R¹ und R² oder R¹ und R⁴ oder R³ und R⁴ gebildet, wobei der Ring insgesamt bevorzugt 5, 6 oder 7 Ringatome aufweist.

Im Sinne der vorliegenden Erfindung bezieht sich der Begriff Esterimid auf Verbindungen der allgemeinen Formel (III) 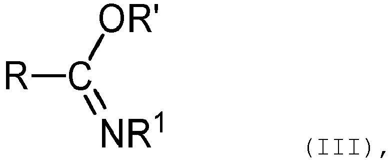 wobei
R' ausgewählt wird aus der Gruppe bestehend aus (C₁-C₂₄)-Alkyl, (C₃-C₂₀)-Cycloalkyl, (C₂-C₁₃) -Heterocycloalkyl;
R ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃)-Heteroaryl, CN, COO-(C₁-C₁₈)-Alkyl, CONH-(C₁-C₁₈)-Alkyl, CF₃;
R¹ ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃)-Heteroaryl;
wobei auch aus jeweils zwei Resten ausgewählt aus R, R¹ und R' gemeinsam eine (C₂-C₈)-Alkylen-Brücke gebildet werden kann, so dass ein Ring mit insgesamt 4-11 Ringatomen entsteht.
Der Begriff Esterimid im Sinne der vorliegenden Erfindung umfasst nicht die Hydrochloride der Esterimide.
R ist bevorzugt ausgewählt aus der Gruppe bestehend aus H, Methyl, Ethyl, n-Propyl, n-Butyl, iso-Butyl, n-Pentyl, n-Hexyl, Cyclohexyl, n-Heptyl, n-Octyl, Phenyl, Pyridyl, Naphthyl.
R' ist bevorzugt ausgewählt aus der Gruppe bestehend aus Methyl, Ethyl, und Benzyl;
R¹ ist bevorzugt ausgewählt aus der Gruppe bestehend aus Wasserstoff, Methyl, Ethyl, n-Propyl, iso-Propyl, n-Butyl, iso-Butyl, n-Hentyl, n-Hexyl, n-Octyl, Benzyl und Phenyl. Ein Ring wird bevorzugt zwischen den Resten R und R¹ oder R¹ und R' gebildet, wobei der Ring insgesamt bevorzugt 5, 6 oder 7 Ringatome aufweist.

Unter einem (C₁-Cₙ)-Alkylrest sind sowohl lineare als auch verzweigte Alkylreste mit 1 bis n C-Atomen zu verstehen. Bei verzweigten Alkylresten kann die Verzweigung an einem beliebigen Kohlenstoff-Atom auftreten.
Bevorzugte (C₁-Cₙ)-Alkylreste sind Methyl, Ethyl, n-Propyl, iso-Propyl, n-Butyl, iso-Butyl, sec-Butyl, n-Pentyl, isoPentyl, n-Hexyl, n-Heptyl, n-Octyl, n-Nonyl, n-Decyl, n-Dodecyl und n-Octadecyl.
Der (C₁-Cₙ)-Alkylrest kann substituiert oder unsubstituiert sein.

Ein (C₃-Cₙ)-Cycloalkylrest bezeichnet ein mono-, bi- oder tricyclisches, aliphatisches System aus insgesamt 3 bis n C-Atomen, wobei jeder Ring drei-, vier-, fünf-, sechs- oder siebengliedrig sein kann. Bevorzugt sind (C₆-C₁₂)-Cycloalkylreste. Besonders bevorzugte (C₃-Cₙ)-Cycloalkylreste sind Cyclopropyl, Cyclobutyl, Cyclopentyl, Cyclohexyl, Cycloheptyl und 1-Adamantyl, 9-Fluorenyl.

Der (C₃-Cₙ)-Cycloalkylrest kann substituiert oder unsubstituiert sein.

Ein (C₂-Cₙ)-Heterocycloalkylrest bezeichnet ein mono-, bi- oder tricyclisches, aliphatisches System aus insgesamt 2 bis n C-Atomen, wobei jeder Ring drei-, vier-, fünf-, sechs- oder siebengliedrig sein kann, und wobei die Anzahl der Heteroatome, ausgewählt aus der Gruppe N, O, und S 1 oder 2 beträgt und die Heteroatome gleich oder verschieden sind. Bevorzugte Heterocycloalkylreste sind 2-, 3-Tetrahydrofuryl, 1-, 2-, 3-Pyrrolidinyl, 1-, 2-, 3-, 4-Piperidinyl, 1-, 2-Piperazinyl, 1-, 2-, 3-Morpholinyl, Tetrahydropyranyl-2 oder -3 und 2,3-Dihydrobenzothiophenyl-2 oder -3.
Der (C₂-Cₙ)-Heterocycloalkylrest kann substituiert oder unsubstituiert sein.

Ein (C₆-Cₙ)-Arylrest bezeichnet ein mono-, bi- und tricyclisches, aromatisches System mit 6 bis n C-Atomen, wobei jeder Ring jeweils fünf-, sechs- oder siebengliedrig sein kann. Bevorzugte (C₆-Cₙ)-Arylreste sind Phenyl, Naphthyl, Anthryl, Phenantryl, Biphenyl.
Der (C₆-Cₙ)-Arylrest kann substituiert oder unsubstituiert sein.

Ein (C₃-Cₙ)-Heteroarylrest bezeichnet ein mono-, bi- oder tricyclisches, aromatisches System aus insgesamt 3 bis n C-Atomen, wobei jeder Ring jeweils fünf-, sechs- oder siebengliedrig sein kann, und wobei die Anzahl der Heteroatome, ausgewählt aus der Gruppe N, O, und S 1 oder 2 beträgt und die Heteroatome gleich oder verschieden sind. Bevorzugte (C₂-Cₙ)-Heteroarylreste sind 2-, 3-Furyl, 2-, 3-Pyrrolyl, 2-, 4-, 5-Imidazolyl, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-, 6-Pyrimidinyl, Acridinyl, Chinolinyl, Phenantridinyl, Benzothienyl.
Der kann substituiert oder unsubstituiert sein.

Substituenten werden ausgewählt aus der Gruppe bestehend aus Halogenen wie F, Cl, Br, I, und heteroatomhaltigen funktionellen Gruppen, die ein oder mehrere Atome ausgewählt aus der Gruppe bestehend aus N, O, P, S, oder Si enthalten, wobei Einfach- und Mehrfachsubstitution möglich ist. Beispiele für heteroatomhaltige funktionelle Gruppen sind Carbonyl-, Carboxyl-, Sulphonat-, Phosphonat-, Hydroxyl-, Silyl-, Amino-, Ammoniumgruppen wie
-OH,
-(C₁-C₈)-Alkyloxy
-COOH,
-NH({C₁-C₈}-Acyl),
-NH({C₁-C₈}-Acyloxy)
-N((C₁-C₂₀)-Alkyl)({C₁-C₈)-Acyl),
-N({C₆-C₁₄}-Aryl)({C₁-C₈}-Acyl),
-N({C₆-C₁₄}-Aralkyl)({C₁-C₈}-Acyl),
-N({C₁-C₈}-Acyl)₂,
-NH₃⁺,
-NH({C₁-C₂₀}-Alkyl)₂⁺,
-NH({C₆-C₁₄}-Aryl)₂⁺,
-NH({C₆-C₁₄}-Aralkyl)₂⁺,
-NH({C₁-C₂₀}-Alkyl)({C₆-C₁₄}-Aryl)⁺,
-N({C₆-C₁₄}-Aryl)({C₁-C₂₀}-Alkyl)⁺,
-N({C₆-C₁₄}-Aryl)₂({C₁-C₂₀}-Alkyl)⁺,
-O-C(=O)-O-{C₁-C₂₀}-Alkyl,
-O-C(=O)-O-{C₆-C₁₄}-Aryl,
-O-C(=O)-O-{C₆-C₁₄}-Aralkyl,
-NH-C(=O)-O-{C₁-C₂₀}-Alkyl,
-NH-C(=O)-O-{C₆-C₁₄}-Aryl,
-NH-C(=O)-O-{C₆-C₁₄}-Aralkyl,
-O-C(=O)-NH-{C₁-C₂₀}-Alkyl,
-O-C(=O)-NH-{C₆-C₁₄}-Aryl,
-O-C(=O)-NH-{C₆-C₁₄}-Aralkyl,
-CN,
-SO₂-O-{C₁-C₂₀} -Alkyl,
-SO₂-O-{C₆-C₁₄}-Aryl,
-SO₂-O-{C₆-C₁₄}-Aralkyl,
-SO₂-{C₁-C₂₀}-Alkyl,
-SO₂-{C₆-C₁₄}-Aryl,
-SO₂-{C₆-C₁₄}-Aralkyl,
-SO-{C₁-C₂₀}-Alkyl,
-SO-{C₆-C₁₄}-Aryl,
-SO-{C₆-C₁₄}-Aralkyl,
-Si({C₁-C₂₀}-Alkyl)₃,
-Si({C₆-C₁₄}-Aryl)₃,
-Si({C₆-C₁₄}-Aryl)({C₁-C₂₀}-Alkyl)₂,
-Si({C₆-C₁₄}-Aryl)₂({C₁-C₂₀}-Alkyl),
-{C₁-C₂₀}-Perfluoroalkyl,
-PO(O-{C₁-C₂₀}-Alkyl)₂,
-PO(O-{C₆-C₁₄}-Aryl)₂,
-PO(O-{C₁-C₂₀}-Alkyl) (O-{C₆-C₁₄}-Aryl),
-PO({C₁-C₂₀}-Alkyl)₂,
-PO({C₆-C₁₄}-Aryl)₂,
-PO({C₁-C₂₀}-Alkyl)({C₆-C₁₄}-Aryl).

Im Sinne der vorliegenden Erfindung ist (C₁-Cₙ)-Alkyloxy definiert als lineare oder verzweigte (C₁-Cₙ)-Alkylgruppe mit 1 bis n C-Atomen mit der Maßgabe, dass diese über ein Sauerstoffatom an das diese Gruppe tragende Molekül gebunden ist.

Im Sinne der vorliegenden Erfindung ist (C₁-Cₙ)-Acyl definiert als eine Gruppe mit der allgemeinen Struktur R-(C=O)- mit insgesamt 1 bis n Kohlenstoffatomen, wobei R ausgewählt wird aus der Gruppe bestehend aus H, (C₁-Cₙ₋₁)-Alkyl, (C₁-Cₙ₋₁)-Alkenyl, (C₆-Cₙ₋₁)-Aryl, (C₆-Cₙ₋₁)-Heteroaryl und (C₂-Cₙ₋₁)-Alkinyl.

Im Sinne der vorliegenden Erfindung ist (C₁-Cₙ)-Acyloxy eine Gruppe mit der allgemeinen Struktur R'-(C=O)O- mit insgesamt 1 bis n Kohlenstoffatomen, wobei R' ausgewählt wird aus der Gruppe bestehend aus H, (C₁-Cₙ₋₁)-Alkyl, (C₁-Cₙ₁)-Alkenyl, (C₆-Cₙ₋₁)-Aryl, (C₆-Cₙ₋₁)-Heteroaryl und (C₂-Cₙ₋₁)-Alkinyl.

Im Sinne der vorliegenden Erfindung ist (C₂-Cₙ)-Alkenyl definiert als lineare oder verzweigte (C₂-Cₙ)-Alkylgruppe mit 2 bis n Kohlenstoffatomen mit der Maßgabe, dass diese eine C-C-Doppelbindung aufweist.

Im Sinne der vorliegenden Erfindung ist (C₂-Cₙ)-Alkinyl definiert als lineare oder verzweigte (C₂-Cₙ)-Alkylgruppe mit 2 bis n Kohlenstoffatomen mit der Maßgabe, dass diese eine C-C-Dreifachbindung aufweist.

Im Sinne der vorliegenden Erfindung bezeichnet (C₆-Cₙ)-Aralkyl
eine Gruppe, die sowohl eine Alkyl- als auch eine Arylgruppe
enthält und in Summe 6 bis n Kohlenstoffatome aufweist. Die Aralkylgruppe kann über jedes ihrer Kohlenstoffatome an das diese Gruppe tragende Molekül gebunden sein. Eine (C₆-Cₙ)-Aralkylgruppe kann auch mit wenigstens einem Substituenten substitutiert sein, wobei die Substituenten unabhängig voneinander ausgewählt werden aus der Gruppe bestehend aus Halogen, (C₁-C₈)-Alkyl, (C₁-C₈)-Alkyloxy, -NH₂, -NO, -NO₂, NH(C₁-C₈)-Alkyl, -N( (C₁-C₈)-Alkyl)₂, -OH, -CF₃, -CₙF₂ₙ₊₁ (wobei n eine ganze Zahl von 2 bis 5 ist), NH(C₁-C₈)-Acyl, -N((C₁-C₈)-Acyl)₂, (C₁-C₈)-Acyl, (C₁-C₈)-Acyloxy, -SO₂- (C₁-C₈) -Alkyl, -SO₂- (C₆-C₁₄) - Aryl, -SO- (C₁-C₈)-Alkyl, -SO-(C₆-C₁₄)-Aryl, -PO(O-{C₁-C₂₀}-Alkyl)₂, -PO(O-{C₆-C₁₄}-Aryl)₂, -PO(O-{C₁-C₂₀}-Alkyl)(O-{C₆-C₁₄}-Aryl), -PO({C₁-C₂₀}-Alkyl)₂, -PO({C₆-C₁₄}-Aryl)₂, -PO({C₁-C₂₀}-Alkyl)({C₆-C₁₄}-Aryl).

Als Hydrierkatalysator können alle dem Fachmann für diesen Zweck in Frage kommenden Hydrierkatalysatoren ausgewählt werden. Bevorzugt werden solche Hydrierkatalysatoren eingesetzt, die mindestens ein aktives Metall enthalten. Bevorzugt ist das aktive Metall eines der Gruppen VII B und/oder VIII B des Periodensystems der Elemente, wobei Edelmetalle und Ni bevorzugt sind, insbesondere sind Ru, Rh, Pd, Pt, Re und Ni bevorzugt. Die Metalle können im Hydrierkatalysator entweder (a) als solche oder in Form von Oxiden oder (b) als Metallkomplexe vorliegen.
In Fall (a) kann das Metall oder Metalloxid entweder auf einem Träger aufgebracht oder als Partikel eingesetzt werden. Das Trägermaterial ist nicht beschränkt, üblicherweise werden gewöhnliche Träger wie Aluminiumoxid, Siliciumdioxid, Aluminiumoxid, Eisenoxid, Magnesiumoxid, Zirkoniumdioxid, Kohlenstoff oder ähnliche dem Fachmann auf dem Gebiet der Hydrierung bekannte Träger eingesetzt. Der Gehalt an Metall oder Metalloxid auf dem Träger wird in einem Bereich von 1 Gew.-% bis 25 Gew.-% bezogen auf das Gesamtgewicht des Katalysators gewählt. Bevorzugt wird ein Gehalt von 1 bis 5 Gew.-% Metall oder Metalloxid auf dem Träger gewählt.
Beispiele für derartige Hydrierkatalysatoren sind Pt/C, Pd/C, Rh/C, Ru/C, Pd/CaCO₃, Pd/Al₂O₃, Ru/Al₂O₃, Rh/Al₂O₃, Pd/Re/C, Pt/Re/C, RuO₂.
In Fall (b) können die Metalle auch in Form von MetallKomplexen als Hydrierkatalysatoren eingesetzt werden. Beispiele hierfür sind Metall-Komplexe der Metalle Rh, Ir oder Ru, wie z.B. der Wilkinson-Katalysator ClRh(PPh₃)₃ oder [(dppb)Rh(cod)]BF4, [Ir(PCy₃(C₅H₅N)(cod)]PF₆, [Cl₂Ru(PPh₃)₃] und [(dppb)Ru(metallyl)₂].
Bevorzugt wird der Hydrierkatalysator ausgewählt aus der Gruppe bestehend aus Pd/C, Pd/Al₂O₃, Pd/CaCO₃, Pt/C, Ru/Al₂O₃, Pd/Re/C, Pt/Re/C und [(dppb)Rh(cod)]BF₄. Besonders bevorzugt sind 5%Pd/C, 5%Pd/Al₂O₃, 5%Pd/CaCO₃, 5%Pt/C, 5%Ru/Al₂O₃, und [(dppb)Rh(cod)]BF₄.
Die Menge des Hydrierkatalysators kann vom Fachmann frei gewählt werden, wobei das Mol-Verhältnis von Hydrierkatalysator zu Amidacetal oder Keten-N,O-acetal oder Esterimid in einem Bereich von 1:10 bis 1:100.000 liegt. Weiter bevorzugt ist ein Bereich von 1:20 bis 1:10.000, besonders bevorzugt ist ein Bereich von 1:50 bis 1:2.000.

Der Wasserstoffdruck der Reaktion wird in einem Bereich von 0,1 bis 200 bar, vorzugsweise von 0,1 bis 100 bar, und besonders bevorzugt von 0,1 bis 60 bar eingestellt.

Die Temperatur, die während der Reaktion einzustellen ist, kann vom Fachmann bestimmt werden und liegt üblicherweise in einem Bereich von 0°C bis 250°C. Sie sollte so hoch sein, dass die anvisierte Reaktion in ausreichend schneller Zeit abläuft, doch möglichst so niedrig sein, dass das Nebenproduktspektrum bei der erfindungsgemäßen Reaktion so gering wie möglich gehalten werden kann. Bevorzugt wird eine Temperatur aus dem Bereich von 0°C bis 120°C eingestellt. Besonders bevorzugt wird eine Temperatur aus dem Bereich von 10°C bis 100°C eingestellt, ganz besonders bevorzugt wird eine Temperatur aus dem Bereich von 20°C bis 50°C eingestellt.

Prinzipiell ist der Fachmann frei in der Wahl des Lösungsmittels, das er in dem erfindungsgemäßen Verfahren einsetzen möchte. Aufgrund der Tatsache, dass die Ausgangsstoffe häufig in flüssiger Form vorliegen, kann insofern auf den Einsatz eines Lösungsmittels auch verzichtet werden. Wenn jedoch der Einsatz von Lösungsmitteln in dem erfindungsgemäßen Verfahren gewünscht ist, ist es vorteilhaft, solche Lösungsmittel heranzuziehen, die die eingesetzten Komponenten der Reaktion entsprechend gut lösen und sich im Übrigen gegenüber der erfindungsgemäßen Reaktion als inert erweisen. Als solche kommen polare oder unpolare Lösungsmittel, insbesondere u.a. Kohlenwasserstoffe, Chlorkohlenwasserstoffe, Ether, Ester und Alkohole in Betracht. Bevorzugt sind dabei Alkane, Halogenalkane, einwertige und mehrwertige Alkohole, cyclische und acyclische Ether, und Ester.
Bevorzugte Lösungsmittel sind solche ausgewählt aus der Gruppe bestehend aus Hexan, Heptan, Octan, Dimethylglykolether (DMGE), 1,4-Dioxan, Methyl-tert-butylether (MTBE), Tetradydrofuran (THF), Essigsäureethylester, Essigsäureisopropyl-ester, Dibutylether, Dimethylcarbonat, Diethylcarbonat, Dipropylcarbonat, Methanol, Ethanol, Isopropanol, Butanol, Ethylenglycol, Dichlormethan, 1,2-Dichlorethan. Besonders bevorzugt sind Methanol und Ethanol.

Es ist vorteilhaft wasserfrei zu arbeiten, so dass bevorzugt wasserfreies Lösungsmittel eingesetzt wird.

Zur Herstellung von Amidacetalen, Keten-N,O-acetalen und Esterimiden können außerdem alle dem Fachmann für diesen Zweck in Frage kommenden Reaktionen ausgewählt werden. Amidacetale können z.B. durch den Austausch von Aminen und Acetalen generiert werden, während Esterimide z.B. durch Alkoholyse von Chloriminen generiert werden können.

Eine besondere Ausführungsform der Erfindung ist ein Verfahren zur Herstellung von Aminen, wobei der Hydrierkatalysator mindestens ein aktives Metall enthält.

Eine besondere Ausführungsform der Erfindung ist ein Verfahren zur Herstellung von Aminen, wobei der Hydrierkatalysator mindestens ein aktives Metall der Gruppe VII B und/oder VIII B des Periodensystems der Elemente enthält.

Eine besondere Ausführungsform der Erfindung ist ein Verfahren zur Herstellung von Aminen, wobei die Umsetzung in einem Lösungsmittel durchgeführt wird.

Eine besondere Ausführungsform der Erfindung ist ein Verfahren zur Herstellung von Aminen, wobei das Lösungsmittel ausgewählt wird aus der Gruppe bestehend aus Kohlenwasserstoffen, Chlorkohlenwasserstoffen, Ethern, Estern und Alkoholen.

Eine besondere Ausführungsform der Erfindung ist ein Verfahren zur Herstellung von Aminen, wobei wasserfreies Lösungsmittel eingesetzt wird.

Eine weitere besondere Ausführungsform der Erfindung ist ein Verfahren zur Herstellung von Aminen, wobei die Umsetzung ohne Lösungsmittel durchgeführt wird.

Ein weiterer Gegenstand der Erfindung ist ein Amin erhältlich durch ein Verfahren umfassend die folgenden Schritte:
a. Umsetzung eines
   i. Amidacetals der allgemeinen Formel (I), 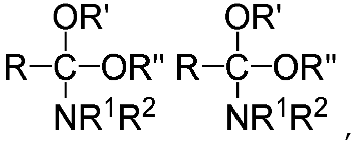 wobei
      R ausgewählt wird aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃)-Heteroaryl, CN, COO-(C₁-C₁₈)-Alkyl, CONH-(C₁-C₁₈)-Alkyl, CF₃;
      R' und R" unabhängig voneinander ausgewählt werden aus der Gruppe (C₁-C₂₄)-Alkyl, (C₃-C₂₀)-Cycloalkyl, (C₂-C₁₃) -Heterocycloalkyl; und
      R¹ und R² unabhängig voneinander ausgewählt werden aus der Gruppe bestehend aus H, (C₁-C₂₄)-Alkyl, (C₃-C₈)-Cycloalkyl, (C₂-C₇)-Heterocycloalkyl, (C₆-C₁₄) -Aryl und (C₃-C₁₃)-Heteroaryl;
      wobei auch aus jeweils zwei Resten ausgewählt aus R, R¹, R², R' und R" gemeinsam eine (C₂-C₈)-Alkylen-Brücke gebildet werden kann, so dass ein Ring mit insgesamt 4-11 Ringatomen entsteht;
         oder
   ii. Keten-N,O-acetals der allgemeinen Formel (II), 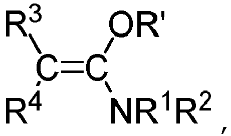 wobei
      R' ausgewählt wird aus der Gruppe bestehend aus (C₁-C₂₄)-Alkyl, (C₃-C₂₀)-Cycloalkyl, (C₂-C₁₃)-Heterocycloalkyl; und
      R¹, R² unabhängig voneinander ausgewählt werden aus der Gruppe bestehend aus H, (C₁-C₂₄)-Alkyl, (C₃-C₂₀)-Cycloalkyl, (C₂-C₁₃)-Heterocycloalkyl, (C₆-C₁₄) -Aryl und (C₃-C₁₃)-Heteroaryl;
      R³ und R⁴ unabhängig voneinander ausgewählt werden aus der Gruppe bestehend aus H, (C₁-C₂₄)-Alkyl, (C₃-C₂₀)-Cycloalkyl, (C₂-C₁₃)-Heterocycloalkyl, (C₆-C₁₄) -Aryl und (C₃-C₁₃)-Heteroaryl, CN, COO-(C₁-C₁₈)-Alkyl, CONH-(C₁-C₁₈)-Alkyl und CF₃, wobei auch aus jeweils zwei Resten ausgewählt aus R¹, R², R³, R⁴ und R' gemeinsam eine (C₂-C₈)-Alkylen-Brücke gebildet werden kann, so dass ein Ring mit insgesamt 3-11 Ringatomen entsteht;
         oder
   iii. Esterimids der allgemeinen Formel (III) 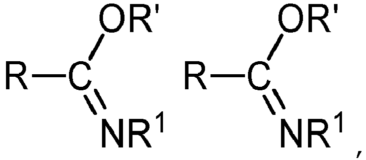 wobei
      R' ausgewählt wird aus der Gruppe bestehend aus (C₁-C₂₄)-Alkyl, (C₃-C₂₀)-Cycloalkyl, (C₂-C₁₃)-Heterocycloalkyl;
      R ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃)-Heteroaryl, CN, COO-(C₁-C₁₈)-Alkyl, CONH-(C₁-C₁₈)-Alkyl, CF₃;
      R¹ ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃)-Heteroaryl;
      wobei auch aus jeweils zwei Resten ausgewählt aus R, R¹ und R' gemeinsam eine (C₂-C₈)-Alkylen-Brücke gebildet werden kann, so dass ein Ring mit insgesamt 4-11 Ringatomen entsteht;
mit H₂ in Gegenwart eines Hydrierkatalysators, wobei Katalysator und Amidacetal oder Keten-N,O-acetal oder Esterimid in einem Mol-Verhältnis von 1:10 bis 1:100.000 eingesetzt werden und wobei ein Wasserstoffdruck von 0,1 bar bis 200 bar eingestellt wird und wobei eine Temperatur im Bereich von 0°C bis 250°C eingestellt wird.

Bevorzugte Reaktionsbedingungen bei der Hydrierung können Tabelle 1 entnommen werden.

**Tabelle 1: Bevorzugte Reaktionsbedingungen bei der Hydrierung.**

| Druck | Temperatur | Hydrierkatalysator | Lösungsmittel | Mol-Verhältnis Katalysator : Edukt |
|---|---|---|---|---|
| 0,1-200 | 0°C-250°C | [a] | ja | 1:10-1:100.000 |
| 0,1-200 | 0°C-250°C | [a] | nein | 1:10-1:100.000 |
| 0,1-200 | 0°C-120°C | [a] | ja | 1:10-1:100.000 |
| 0,1-200 | 0°C-120°C | [a] | nein | 1:10-1:100.000 |
| 0,1-200 | 10°C-100°C | [a] | ja | 1:10-1:100.000 |
| 0,1-200 | 10°C-100°C | [a] | nein | 1:10-1:100.000 |
| 0,1-100 | 0°C-250°C | [a] | ja | 1:10-1:100.000 |
| 0,1-100 | 0°C-250°C | [a] | nein | 1:10-1:100.000 |
| 0,1-100 | 0°C-120°C | [a] | ja | 1:10-1:100.000 |
| 0,1-100 | 0°C-120°C | [a] | nein | 1:10-1:100.000 |
| 0,1-100 | 10°C-100°C | [a] | ja | 1:10-1:100.000 |
| 0,1-100 | 10°C-100°C | [a] | nein | 1:10-1:100.000 |
| 0,1-60 | 0°C-250°C | [a] | ja | 1:10-1:100.000 |
| 0,1-60 | 0°C-250°C | [a] | nein | 1:10-1:100.000 |
| 0,1-60 | 0°C-120°C | [a] | ja | 1:10-1:100.000 |
| 0,1-60 | 0°C-120°C | [a] | nein | 1:10-1:100.000 |
| 0,1-60 | 10°C-100°C | [a] | ja | 1:10-1:100.000 |
| 0,1-60 | 10°C-100°C | [a] | nein | 1:10-1:100.000 |
| 40 bar H₂ | 20°C-50°C | [a] | ja | 1:10-1:100.000 |
| 40 bar H₂ | 20°-50°C | [a] | nein | 1:10-1:100.000 |
| 0,1-200 | 0°C-250°C | [b] | ja | 1:10-1:100.000 |
| 0,1-200 | 0°C-250°C | [b] | nein | 1:10-1:100.000 |
| 0,1-200 | 0°C-120°C | [b] | ja | 1:10-1:100.000 |
| 0,1-200 | 0°C-120°C | [b] | nein | 1:10-1:100.000 |
| 0,1-200 | 10°C-100°C | [b] | ja | 1:10-1:100.000 |
| 0,1-200 | 10°C-100°C | [b] | nein | 1:10-1:100.000 |
| 0,1-100 | 0°C-250°C | [b] | ja | 1:10-1:100.000 |
| 0,1-100 | 0°C-250°C | [b] | nein | 1:10-1:100.000 |
| 0,1-100 | 0°C-120°C | [b] | ja | 1:10-1:100.000 |
| 0,1-100 | 0°C-120°C | [b] | nein | 1:10-1:100.000 |
| 0,1-100 | 10°C-100°C | [b] | ja | 1:10-1:100.000 |
| 0,1-100 | 10°C-100°C | [b] | nein | 1:10-1:100.000 |
| 0,1-60 | 0°C-250°C | [b] | ja | 1:10-1:100.000 |
| 0,1-60 | 0°C-250°C | [b] | nein | 1:10-1:100.000 |
| 0,1-60 | 0°C-120°C | [b] | ja | 1:10-1:100.000 |
| 0,1-60 | 0°C-120°C | [b] | nein | 1:10-1:100.000 |
| 0,1-60 | 10°C-100°C | [b] | ja | 1:10-1:100.000 |
| 0,1-60 | 10°C-100°C | [b] | nein | 1:10-1:100.000 |
| 40 bar H₂ | 20°C-50°C | [b] | ja | 1:10-1:100.000 |
| 40 bar H₂ | 20°-50°C | [b] | nein | 1:10-1:100.000 |
| 0,1-200 | 0°C-250°C | [c] | ja | 1:10-1:100.000 |
| 0,1-200 | 0°C-250°C | [c] | nein | 1:10-1:100.000 |
| 0,1-200 | 0°C-120°C | [c] | ja | 1:10-1:100.000 |
| 0,1-200 | 0°C-120°C | [c] | nein | 1:10-1:100.000 |
| 0,1-200 | 10°C-100°C | [c] | ja | 1:10-1:100.000 |
| 0,1-200 | 10°C-100°C | [c] | nein | 1:10-1:100.000 |
| 0,1-100 | 0°C-250°C | [c] | ja | 1:10-1:100.000 |
| 0,1-100 | 0°C-250°C | [c] | nein | 1:10-1:100.000 |
| 0,1-100 | 0°C-120°C | [c] | ja | 1:10-1:100.000 |
| 0,1-100 | 0°C-120°C | [c] | nein | 1:10-1:100.000 |
| 0,1-100 | 10°C-100°C | [c] | ja | 1:10-1:100.000 |
| 0,1-100 | 10°C-100°C | [c] | nein | 1:10-1:100.000 |
| 0,1-60 | 0°C-250°C | [c] | ja | 1:10-1:100.000 |
| 0,1-60 | 0°C-250°C | [c] | nein | 1:10-1:100.000 |
| 0,1-60 | 0°C-120°C | [c] | ja | 1:10-1:100.000 |
| 0,1-60 | 0°C-120°C | [c] | nein | 1:10-1:100.000 |
| 0,1-60 | 10°C-100°C | [c] | ja | 1:10-1:100.000 |
| 0,1-60 | 10°C-100°C | [c] | nein | 1:10-1:100.000 |
| 40 bar H₂ | 20°C-50°C | [c] | ja | 1:10-1:100.000 |
| 40 bar H₂ | 20°-50°C | [c] | nein | 1:10-1:100.000 |
| 0,1-200 | 0°C-250°C | [a] | ja | 1:20-1:10.000 |
| 0,1-200 | 0°C-250°C | [a] | nein | 1:20-1:10.000 |
| 0,1-200 | 0°C-120°C | [a] | ja | 1:20-1:10.000 |
| 0,1-200 | 0°C-120°C | [a] | nein | 1:20-1:10.000 |
| 0,1-200 | 10°C-100°C | [a] | ja | 1:20-1:10.000 |
| 0,1-200 | 10°C-100°C | [a] | nein | 1:20-1:10.000 |
| 0,1-100 | 0°C-250°C | [a] | ja | 1:20-1:10.000 |
| 0,1-100 | 0°C-250°C | [a] | nein | 1:20-1:10.000 |
| 0,1-100 | 0°C-120°C | [a] | ja | 1:20-1:10.000 |
| 0,1-100 | 0°C-120°C | [a] | nein | 1:20-1:10.000 |
| 0,1-100 | 10°C-100°C | [a] | ja | 1:20-1:10.000 |
| 0,1-100 | 10°C-100°C | [a] | nein | 1:20-1:10.000 |
| 0,1-60 | 0°C-250°C | [a] | ja | 1:20-1:10.000 |
| 0,1-60 | 0°C-250°C | [a] | nein | 1:20-1:10.000 |
| 0,1-60 | 0°C-120°C | [a] | ja | 1:20-1:10.000 |
| 0,1-60 | 0°C-120°C | [a] | nein | 1:20-1:10.000 |
| 0,1-60 | 10°C-100°C | [a] | ja | 1:20-1:10.000 |
| 0,1-60 | 10°C-100°C | [a] | nein | 1:20-1:10.000 |
| 40 bar H₂ | 20°C-50°C | [a] | ja | 1:20-1:10.000 |
| 40 bar H₂ | 20°-50°C | [a] | nein | 1:20-1:10.000 |
| 0,1-200 | 0°C-250°C | [b] | ja | 1:20-1:10.000 |
| 0,1-200 | 0°C-250°C | [b] | nein | 1:20-1:10.000 |
| 0,1-200 | 0°C-120°C | [b] | ja | 1:20-1:10.000 |
| 0,1-200 | 0°C-120°C | [b] | nein | 1:20-1:10.000 |
| 0,1-200 | 10°C-100°C | [b] | ja | 1:20-1:10.000 |
| 0,1-200 | 10°C-100°C | [b] | nein | 1:20-1:10.000 |
| 0,1-100 | 0°C-250°C | [b] | ja | 1:20-1:10.000 |
| 0,1-100 | 0°C-250°C | [b] | nein | 1:20-1:10.000 |
| 0,1-100 | 0°C-120°C | [b] | ja | 1:20-1:10.000 |
| 0,1-100 | 0°C-120°C | [b] | nein | 1:20-1:10.000 |
| 0,1-100 | 10°C-100°C | [b] | ja | 1:20-1:10.000 |
| 0,1-100 | 10°C-100°C | [b] | nein | 1:20-1:10.000 |
| 0,1-60 | 0°C-250°C | [b] | ja | 1:20-1:10.000 |
| 0,1-60 | 0°C-250°C | [b] | nein | 1:20-1:10.000 |
| 0,1-60 | 0°C-120°C | [b] | ja | 1:20-1:10.000 |
| 0,1-60 | 0°C-120°C | [b] | nein | 1:20-1:10.000 |
| 0,1-60 | 10°C-100°C | [b] | ja | 1:20-1:10.000 |
| 0,1-60 | 10°C-100°C | [b] | nein | 1:20-1:10.000 |
| 40 bar H₂ | 20°C-50°C | [b] | ja | 1:20-1:10.000 |
| 40 bar H₂ | 20°-50°C | [b] | nein | 1:20-1:10.000 |
| 0,1-200 | 0°C-250°C | [c] | ja | 1:20-1:10.000 |
| 0,1-200 | 0°C-250°C | [c] | nein | 1:20-1:10.000 |
| 0,1-200 | 0°C-120°C | [c] | ja | 1:20-1:10.000 |
| 0,1-200 | 0°C-120°C | [c] | nein | 1:20-1:10.000 |
| 0,1-200 | 10°C-100°C | [c] | ja | 1:20-1:10.000 |
| 0,1-200 | 10°C-100°C | [c] | nein | 1:20-1:10.000 |
| 0,1-100 | 0°C-250°C | [c] | ja | 1:20-1:10.000 |
| 0,1-100 | 0°C-250°C | [c] | nein | 1:20-1:10.000 |
| 0,1-100 | 0°C-120°C | [c] | ja | 1:20-1:10.000 |
| 0,1-100 | 0°C-120°C | [c] | nein | 1:20-1:10.000 |
| 0,1-100 | 10°C-100°C | [c] | ja | 1:20-1:10.000 |
| 0,1-100 | 10°C-100°C | [c] | nein | 1:20-1:10.000 |
| 0,1-60 | 0°C-250°C | [c] | ja | 1:20-1:10.000 |
| 0,1-60 | 0°C-250°C | [c] | nein | 1:20-1:10.000 |
| 0,1-60 | 0°C-120°C | [c] | ja | 1:20-1:10.000 |
| 0,1-60 | 0°C-120°C | [c] | nein | 1:20-1:10.000 |
| 0,1-60 | 10°C-100°C | [c] | ja | 1:20-1:10.000 |
| 0,1-60 | 10°C-100°C | [c] | nein | 1:20-1:10.000 |
| 40 bar H₂ | 20°C-50°C | [c] | ja | 1:20-1:10.000 |
| 40 bar H₂ | 20°-50°C | [c] | nein | 1:20-1:10.000 |
| 0,1-200 | 0°C-250°C | [a] | ja | 1:50-1:2.000 |
| 0,1-200 | 0°C-250°C | [a] | nein | 1:50-1:2.000 |
| 0,1-200 | 0°C-120°C | [a] | ja | 1:50-1:2.000 |
| 0,1-200 | 0°C-120°C | [a] | nein | 1:50-1:2.000 |
| 0,1-200 | 10°C-100°C | [a] | ja | 1:50-1:2.000 |
| 0,1-200 | 10°C-100°C | [a] | nein | 1:50-1:2.000 |
| 0,1-100 | 0°C-250°C | [a] | ja | 1:50-1:2.000 |
| 0,1-100 | 0°C-250°C | [a] | nein | 1:50-1:2.000 |
| 0,1-100 | 0°C-120°C | [a] | ja | 1:50-1:2.000 |
| 0,1-100 | 0°C-120°C | [a] | nein | 1:50-1:2.000 |
| 0,1-100 | 10°C-100°C | [a] | ja | 1:50-1:2.000 |
| 0,1-100 | 10°C-100°C | [a] | nein | 1:50-1:2.000 |
| 0,1-60 | 0°C-250°C | [a] | ja | 1:50-1:2.000 |
| 0,1-60 | 0°C-250°C | [a] | nein | 1:50-1:2.000 |
| 0,1-60 | 0°C-120°C | [a] | ja | 1:50-1:2.000 |
| 0,1-60 | 0°C-120°C | [a] | nein | 1:50-1:2.000 |
| 0,1-60 | 10°C-100°C | [a] | ja | 1:50-1:2.000 |
| 0,1-60 | 10°C-100°C | [a] | nein | 1:50-1:2.000 |
| 40 bar H₂ | 20°C-50°C | [a] | ja | 1:50-1:2.000 |
| 40 bar H₂ | 20°-50°C | [a] | nein | 1:50-1:2.000 |
| 0,1-200 | 0°C-250°C | [b] | ja | 1:50-1:2.000 |
| 0,1-200 | 0°C-250°C | [b] | nein | 1:50-1:2.000 |
| 0,1-200 | 0°C-120°C | [b] | ja | 1:50-1:2.000 |
| 0,1-200 | 0°C-120°C | [b] | nein | 1:50-1:2.000 |
| 0,1-200 | 10°C-100°C | [b] | ja | 1:50-1:2.000 |
| 0,1-200 | 10°C-100°C | [b] | nein | 1:50-1:2.000 |
| 0,1-100 | 0°C-250°C | [b] | ja | 1:50-1:2.000 |
| 0,1-100 | 0°C-250°C | [b] | nein | 1:50-1:2.000 |
| 0,1-100 | 0°C-120°C | [b] | ja | 1:50-1:2.000 |
| 0,1-100 | 0°C-120°C | [b] | nein | 1:50-1:2.000 |
| 0,1-100 | 10°C-100°C | [b] | ja | 1:50-1:2.000 |
| 0,1-100 | 10°C-100°C | [b] | nein | 1:50-1:2.000 |
| 0,1-60 | 0°C-250°C | [b] | ja | 1:50-1:2.000 |
| 0,1-60 | 0°C-250°C | [b] | nein | 1:50-1:2.000 |
| 0,1-60 | 0°C-120°C | [b] | ja | 1:50-1:2.000 |
| 0,1-60 | 0°C-120°C | [b] | nein | 1:50-1:2.000 |
| 0,1-60 | 10°C-100°C | [b] | ja | 1:50-1:2.000 |
| 0,1-60 | 10°C-100°C | [b] | nein | 1:50-1:2.000 |
| 40 bar H₂ | 20°C-50°C | [b] | ja | 1:50-1:2.000 |
| 40 bar H₂ | 20°-50°C | [b] | nein | 1:50-1:2.000 |
| 0,1-200 | 0°C-250°C | [c] | ja | 1:50-1:2.000 |
| 0,1-200 | 0°C-250°C | [c] | nein | 1:50-1:2.000 |
| 0,1-200 | 0°C-120°C | [c] | ja | 1:50-1:2.000 |
| 0,1-200 | 0°C-120°C | [c] | nein | 1:50-1:2.000 |
| 0,1-200 | 10°C-100°C | [c] | ja | 1:50-1:2.000 |
| 0,1-200 | 10°C-100°C | [c] | nein | 1:50-1:2.000 |
| 0,1-100 | 0°C-250°C | [c] | ja | 1:50-1:2.000 |
| 0,1-100 | 0°C-250°C | [c] | nein | 1:50-1:2.000 |
| 0,1-100 | 0°C-120°C | [c] | ja | 1:50-1:2.000 |
| 0,1-100 | 0°C-120°C | [c] | nein | 1:50-1:2.000 |
| 0,1-100 | 10°C-100°C | [c] | ja | 1:50-1:2.000 |
| 0,1-100 | 10°C-100°C | [c] | nein | 1:50-1:2.000 |
| 0,1-60 | 0°C-250°C | [c] | ja | 1:50-1:2.000 |
| 0,1-60 | 0°C-250°C | [c] | nein | 1:50-1:2.000 |
| 0,1-60 | 0°C-120°C | [c] | ja | 1:50-1:2.000 |
| 0,1-60 | 0°C-120°C | [c] | nein | 1:50-1:2.000 |
| 0,1-60 | 10°C-100°C | [c] | ja | 1:50-1:2.000 |
| 0,1-60 | 10°C-100°C | [c] | nein | 1:50-1:2.000 |
| 40 bar H₂ | 20°C-50°C | [c] | ja | 1:50-1:2.000 |
| 40 bar H₂ | 20°-50°C | [c] | nein | 1:50-1:2.000 |

| | | | | |
|---|---|---|---|---|
| [a] = Hydrierkatalysator mit mindestens einem aktiven Metall der Gruppe VII B und/oder VIII B des Periodensystems der Elemente [b] = Pd/C, Pd/Al₂O₃, Pd/CaCO₃, Pt/C, Ru/Al₂O₃, Pd/Re/C, Pt/Re/C oder [(dppb)Rh(cod)]BF₄ [c] = 5%Pd/C, 5%Pd/Al₂O₃, 5%Pd/CaCO₃, 5%Pt/C, 5%Ru/Al₂O₃ oder [(dppb)Rh(cod)]BF₄ | | | | |

Bei dem erfindungsgemäßen Verfahren geht man im Allgemeinen so vor, dass man in einem Autoklaven das Amidacetal oder Esterimid oder Keten-N,O-acetal und den Hydrierkatalysator in dem genannten Mol-Verhältnis mit einer geeigneten Menge Lösungsmittel vermischt. Anschließend wird der Autoklav mehrmals mit Wasserstoff gespült und die Mischung bei geeignetem Druck hydriert. Nachdem der Wasserstoffdruck abgelassen wurde, wird die Reaktionsmischung abfiltriert und das Filtrat nach dem Fachmann bekannten Verfahren aufgearbeitet.

### Ausführungsbeispiele

Die nachstehenden Beispiele dienen zur Erläuterung der Erfindung, ohne sie darauf zu beschränken.

### Beispiel 1 (Wiederholung des Versuchs 1 aus der Patentschrift DE-604277 (1934)):

In einem 300 ml Autoklav wird Benzyliminoethylether Hydrochlorid (37 g, 0.2 mol) in 30 mL gekühltem absolutem Ethanol suspendiert und nach Zugabe von 0.5 g Platinoxid (Adams)-Katalysator mit Wasserstoff gespült, anschließend wurden 40 bar Wasserstoff aufgepresst und bei 30°C und konstantem Druck 12 Stunden gerührt. Nach Abfiltrieren vom Katalysator und Abdestillieren des Lösungsmittels wurde der Rückstand mit 50 ml 2N Natronlauge versetzt, und das Produkt wird mit Diethylether extrahiert. Die organische Phase wird über K₂CO₃ getrocknet, das Lösemittel wird am Rotationsverdampfer entfernt, und der Rückstand wird mittels GC-MS und NMR Spektroskopie analysiert. Das Gemisch besteht aus 2% Benzylamin, 21 % (Cyclohexylmethyl)amin und 77% Di-(cyclohexalmethyl)amin.

### Beispiele 2-19:

In einem 10 ml Autoklav wird ein Amidacetal (5mmol) in 5mL absolutem Methanol gelöst, nach Zugabe von 1 mol% Katalysator wird mit Wasserstoff gespült. Anschließend werden 40 bar Wasserstoff aufgepresst, und es wird bei 25°C und konstantem Druck gerührt, bis keine Wasserstoffaufnahme mehr zu erkennen ist (1-2 h). Die Reaktionslösung wird nach Abfiltrieren vom Katalysator mit 10 ml 1MHCl Lösung in Methanol versetzt, das Lösemittel wird am Rotationsverdampfer entfernt, der Rückstand wird mit Ether versetzt. Das feste Amin-hydrochlorid wird abfiltriert, mit Diethylether gewaschen und im Vakuum getrocknet.

### Beispiele 20-25:

In einem Autoklav wird 2-Ethoxy-2-methyl-3-benzyl-oxazolidin (5.55 g, 25 mmol) in 25mL absolutem Ethanol gelöst und nach Zugabe von 1 mol% Katalysator mit Wasserstoff gespült. Anschließend werden 40 bar Wasserstoff aufgepresst, und es wird bei 25°C und konstantem Druck gerührt, bis keine Wasserstoffaufnahme mehr zu erkennen ist (2-3 h). Nach Abtrennen vom Katalysator und Destillieren erhält man N-Benzyl-2-(ethylamino)ethanol: Kp 63°C/0.03 mbar; Rf = 0.33 in Ethylacetat; ¹H NMR (CDCl₃) δ7.33-7.37 (m, 4H), 7. 27-7.30 (m, 1H), 3. 66 (s, 2H), 3.60 (t, J = 5.4, 2H), 2.96 (br. s, OH), 2.69 (t, J = 5.4, 2H), 2.61 (q, J = 7.2, 2H), 1.10 (t, J = 7.2, 3H).

Die eingesetzten Katalysatoren und Ausbeuten der Beispiele 2-25 können Tabelle 2 entnommen werden.

**Tabelle 2: Ausbeuten der Beispiele 2-25.**

| Beispiele | 2-7 | 8-13 | 14-19 | 20-25 |
|---|---|---|---|---|
| Amidacetal | 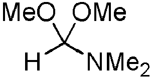 | 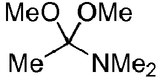 | 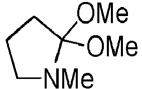 | 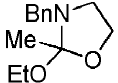 |
| Amin | NMe₃ | EtNMe₂ | 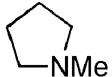 | 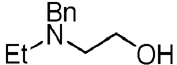 |
| 5%Pd/C | 60 % | 84 % | 74 % | 80 % |
| 5%Pd/Al₂O₃ | 70 % | 99 % | 64 % | 50 % |
| 5%Pd/CaCO₃ | 74 % | 93 % | 69 % | 40 % |
| 5%Pt/C | 78 % | 82 % | 99 % | 82 % |
| 5%Ru/Al₂O₃ | 83 % | 80 % | 76 % | 22 % |
| [(dppb)Rh(cod)]BF₄ | 96 % | 97 % | 95 % | 97 % |

### Beispiele 26-31:

In einem Autoklav wird ein Esterimid (25 mmol) in 25 mL absolutem Methanol oder Ethanol gelöst und nach Zugabe von 5% Pt/C (975 mg, 1 mol%) mit Wasserstoff gespült. Anschließend werden 40 bar Wasserstoff aufgepresst, und es wird bei 25°C und konstantem Druck 16 Stunden gerührt. Nach Abtrennen vom Katalysator wird das Filtrat destilliert.
Die Reaktionsbedingungen und Ausbeuten können Tabelle 3 entnommen werden.

**Tabelle 3: Reaktionsbedingungen und Ausbeuten der Beispiele 26-31.**

| Beispiel | Esterimid | Produkt | Lösemittel | Ausbeute, % |
|---|---|---|---|---|
| 26 | 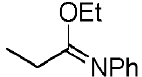 | 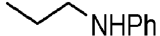 | EtOH | 86 |
| 27 | 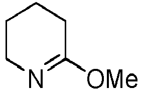 | 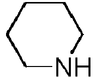 | MeOH | 84 |
| 28 | 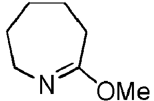 | 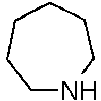 | MeOH | 85 |
| 29 | 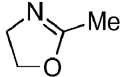 | 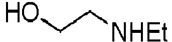 | - | 60 |
| | | 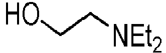 | - | 19 |
| 30 | 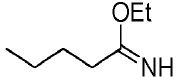 | (C₅H₁₁)₂NH | EtOH | 88 |
| 31 | 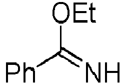 | Bn₂NH | EtOH | 85 |

### Beispiel 32:

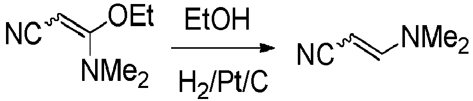

In einem Autoklav wird 2-Dimethylamino-2-ethoxy-1-cyanoethylen (7 g, 50 mmol) in 20 mL absolutem Ethanol gelöst. Nach Zugabe von 5% Pt/C (975 mg, 0.5mol%) wird der Autoklav mit Wasserstoff gespült, anschließend werden 40 bar Wasserstoff aufgepresst, und es wird bei 50°C und konstantem Druck 16 Stunden gerührt. Nach Abfiltrieren vom Katalysator und Abdestillieren des Lösungsmittels wird der Rückstand in Diethylether aufgenommen, mit konzentrierter NaCl-Lösung gewaschen und über MgSO4 getrocknet. Nach Destillieren erhält man 3.1 g (65%) von 3-(Dimethylamino)acrylonitril; Kp = 66°C/0.1mbar, ¹H NMR (CD₂Cl₂) (bevorzugte isomer) δ6.95 (d, J = 13.6, 1H), 3.70 (d, J = 13.5, 1H), 2.86 (br. S, 6H).

## Patentansprüche

1. Verfahren zur Herstellung von Aminen umfassend die folgenden Schritte:
a. Umsetzung eines
i. Amidacetals der allgemeinen Formel (I), 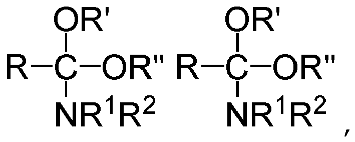 wobei
R ausgewählt wird aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃) -Heteroaryl, CN, COO- (C₁-C₁₈) -Alkyl, CONH- (C₁-C₁₈) -Alkyl, CF₃;
R' und R" unabhängig voneinander ausgewählt werden aus der Gruppe (C₁-C₂₄)-Alkyl, (C₃-C₂₀)-Cycloalkyl, (C₂-C₁₃)-Heterocycloalkyl;
und
R¹ und R² unabhängig voneinander ausgewählt werden aus der Gruppe bestehend aus H, (C₁-C₂₄)-Alkyl, (C₃-C₈)-Cycloalkyl, (C₂-C₇)-Heterocycloalkyl, (C₆-C₁₄) -Aryl und (C₃-C₁₃)-Heteroaryl;
wobei auch aus jeweils zwei Resten ausgewählt aus R, R¹, R², R' und R" gemeinsam eine (C₂-C₈)-Alkylen-Brücke gebildet werden kann, so dass ein Ring mit insgesamt 4-11 Ringatomen entsteht;
oder
ii. Keten-N,O-acetals der allgemeinen Formel (II), 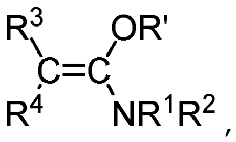 wobei
R' ausgewählt wird aus der Gruppe bestehend aus (C₁-C₂₄)-Alkyl, (C₃-C₂₀)-Cycloalkyl, (C₂-C₁₃)-Heterocycloalkyl; und
R¹, R² unabhängig voneinander ausgewählt werden aus der Gruppe bestehend aus H, (C₁-C₂₄)-Alkyl, (C₃-C₂₀)-Cycloalkyl, (C₂-C₁₃)-Heterocycloalkyl, (C₆-C₁₄) -Aryl und (C₃-C₁₃)-Heteroaryl;
R³ und R⁴ unabhängig voneinander ausgewählt werden aus der Gruppe bestehend aus H, (C₁-C₂₄)-Alkyl, (C₃-C₂₀)-Cycloalkyl, (C₂-C₁₃)-Heterocycloalkyl, (C₆-C₁₄) -Aryl und (C₃-C₁₃)-Heteroaryl, CN, COO-(C₁-C₁₈)-Alkyl, CONH-(C₁-C₁₈)-Alkyl und CF₃,
wobei auch aus jeweils zwei Resten ausgewählt aus R¹, R², R³, R⁴ und R' gemeinsam eine (C₂-C₈)-Alkylen-Brücke gebildet werden kann, so dass ein Ring mit insgesamt 3-11 Ringatomen entsteht; oder
iii. Esterimids der allgemeinen Formel (III) 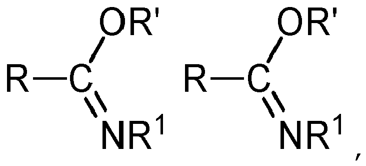 wobei
R' ausgewählt wird aus der Gruppe bestehend aus (C₁-C₂₄)-Alkyl, (C₃-C₂₀)-Cycloalkyl, (C₂-C₁₃)-Heterocycloalkyl;
R ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃)-Heteroaryl, CN, COO-(C₁-C₁₈)-Alkyl, CONH-(C₁-C₁₈)-Alkyl, CF₃;
R¹ ausgewählt aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl oder (C₃-C₁₃)-Heteroaryl;
wobei auch aus jeweils zwei Resten ausgewählt aus R, R¹ und R' gemeinsam eine (C₂-C₈)-Alkylen-Brücke gebildet werden kann, so dass ein Ring mit insgesamt 4-11 Ringatomen entsteht;
mit H₂ in Gegenwart eines Hydrierkatalysators, wobei Katalysator und Amidacetal oder Keten-N,O-acetal oder Esterimid in einem Mol-Verhältnis von 1:10 bis 1:100.000 eingesetzt werden und wobei ein Wasserstoffdruck von 0,1 bar bis 200 bar eingestellt wird und wobei eine Temperatur im Bereich von 0°C bis 250°C eingestellt wird.

2. Verfahren gemäß Anspruch 1, wobei der Hydrierkatalysator mindestens ein aktives Metall enthält.

3. Verfahren gemäß Anspruch 2, wobei das aktive Metall ein Metall der Gruppe VII B und/oder VIII B des Periodensystems der Elemente ist.

4. Verfahren gemäß einem der Ansprüche 1-3, wobei die Umsetzung in einem Lösungsmittel durchgeführt wird.

5. Verfahren gemäß Anspruch 4, wobei das Lösungsmittel ausgewählt wird aus der Gruppe bestehend aus Kohlenwasserstoffen, Chlorkohlenwasserstoffen, Ethern, Estern und Alkoholen.

6. Verfahren gemäß Anspruch 5, wobei wasserfreies Lösungsmittel eingesetzt wird.

7. Verfahren gemäß einem der Ansprüche 1-3, wobei die Umsetzung ohne Lösungsmittel durchgeführt wird.

8. Amin erhältlich durch ein Verfahren gemäß einem der Ansprüche 1-7.
